(19) Europäisches Patentamt / European Patent Office / Office européen des brevets

(11) **EP 2 585 421 B1**

(12) **EUROPEAN PATENT SPECIFICATION**

(45) Date of publication and mention
of the grant of the patent:
**19.09.2018 Bulletin 2018/38**

(21) Application number: **10853775.4**

(22) Date of filing: **23.06.2010**

(51) Int Cl.:
*B01J 37/28* (2006.01)     *B01J 21/06* (2006.01)
*B01J 29/08* (2006.01)     *C07C 1/24* (2006.01)
*C07C 11/06* (2006.01)     *B01J 29/40* (2006.01)
*B01J 29/18* (2006.01)     *B01J 29/46* (2006.01)
*B01J 29/48* (2006.01)     *B01J 29/65* (2006.01)
*B01J 29/70* (2006.01)

(86) International application number:
**PCT/SG2010/000235**

(87) International publication number:
**WO 2011/162717 (29.12.2011 Gazette 2011/52)**

(54) **METHOD OF PRODUCING ALKENES BY DEHYDRATION OF A MIXTURE OF ALCOHOLS**

VERFAHREN ZUR HERSTELLUNG VON ALKENEN MITTELS DEHYDRIERUNG EINER ALKOHOLMISCHUNG

PROCÉDÉ DE PRODUCTION D'ALCÈNES PAR DÉSHYDRATATION D'UN MÉLANGE D'ALCOOLS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**01.05.2013 Bulletin 2013/18**

(73) Proprietor: **Agency for Science, Technology and Research**
**Singapore 138632 (SG)**

(72) Inventors:
• **RAMESH, Kanaparthi**
**Singapore 627833 (SG)**
• **BORGNA, Armando**
**Singapore 627833 (SG)**
• **ZHENG, Jia'E**
**Singapore 627833 (SG)**

(74) Representative: **Viering, Jentschura & Partner mbB**
**Patent- und Rechtsanwälte**
**Kennedydamm 55 / Roßstrasse**
**40476 Düsseldorf (DE)**

(56) References cited:
WO-A1-2007/043731     WO-A1-2009/022990
WO-A2-2007/083241     JP-A- 2010 018 556
US-A- 4 698 452       US-A- 4 698 452
US-A1- 2008 216 391

• **BALCI S: "Ethylene and acetaldehyde production by selective oxidation of ethanol using mesoporous V-MCM-41 catalysts", INDUSTRIAL & ENGINEERING CHEMISTRY RESEARCH, AMERICAN CHEMICAL SOCIETY, US, vol. 45, no. 10, 1 January 2006 (2006-01-01), pages 3496-3502, XP009130500, ISSN: 0888-5885, DOI: 10.1021/IE050952J**
• **DATABASE WPI Derwent Publications Ltd., London, GB; AN 2010-B03926, XP003031734 & CN 101 623 652 A (UNIVNANJINGTECHNOLOGY) 13 January 2010**
• **DATABASE WPI Derwent Publications Ltd., London, GB; AN 2008-N85470, XP003031733 & CN 101 274 285 A (UNIVHUNAN) 01 October 2008**

**Description**

**FIELD OF THE INVENTION**

The invention relates to a method of producing alkenes by dehydration of a mixture of alcohols. In particular, the invention relates to the use of a metal-modified zeolite in said process.

**BACKGROUND OF THE INVENTION**

Light olefins are important intermediates or building blocks in the chemical industry, mainly produced using steam cracking of hydrocarbons. Dwindling petroleum resources combined with its volatile price and environmental concerns are directing the industries to develop alternative technologies based on renewable sources. Propylene is widely used in the chemical industry in the production of iso-propanol, acrylonitrile, acrylic acid, cumene and propylene oxide. But the major application of propylene is in the manufacturing of polypropylene. On the other hand C3+ olefins (i.e. olefins having three or more carbon atoms) can be used as chemical intermediates or fuels. Therefore, the improvement of propylene yield and the development on-purpose processes to produce propylene from alternative feedstocks are important industrial targets.

Propylene demand worldwide is growing faster than ethylene. Propylene is largely produced as a by-product in the thermal steam cracking of liquid feedstock such as naphtha (cf. H. Zimmermann and R. Walzi, Ullmann's Encyclopedia of Industrial Chemistry, 7th Ed. (2010), John Wiley & Sons). However, the yield of propylene from naphtha cracker is about 15 % with propylene to ethylene ratio of about 0.5, depending on the severity of the process. Propylene is also a by-product in the fluid catalytic cracking. Alternatively, propylene also produced from the dehydrogenation of propane (cf. M. Baerns and O. Buyevskaya, Catal. Today 45 (1998) 13). The major challenge in this process is to minimize the formation of carbon oxides (COx) by complete conversion of hydrocarbons. Alternatively propylene with high yields can also be produced from methanol to olefins process using Silicoaluminophosphates (SAPO) and ZSM-5 zeolites as catalysts. Coke formed in the process need to be removed by combustion with air. However, the production of propylene from renewable alcohols such as ethanol and butanol has not been widely reported. Since alcohols can be produced from renewable feedstock, it is clear that the production of propylene from bio-alcohols is an important target.

Currently, bio ethanol is being widely produced via fermentation process from renewable sources such as biomass feed stocks like corn, sugarcane and cellulose. There are mainly two categories of feed stocks for the ethanol fermentation, which are classified as $1^{st}$ generation and $2^{nd}$ generation sources. The $1^{st}$ generation feed stocks are sugars (i.e. sugar beets, sugar canes), starch (i.e. corn, wheat) and oil (i.e. rape seed, soybean) while $2^{nd}$ generation feed stocks are lignocelluloses (i.e. lignin, hemicelluloses, and cellulose), wood chips, crop residues, and tall grasses. The $2^{nd}$ generation sources are preferred mainly because they are not competing with food. Moreover, the feedstock for $2^{nd}$ generation fermentation is usually much cheaper as most of them are agricultural wastes. Ethanol is also a by-product in the sugar cane industry. The ethanol produced from the above processes is considered as renewable feedstock which is independent of fossil fuel, being used as fuels or fuel additives in automotive engines. Alternatively, production of intermediate and value-added chemicals from ethanol is currently attracting considerable interest because of the volatile oil price and concerns for climate change. Dehydration of lower alcohols (methanol and ethanol) derived from natural gas or biomass to olefins represents an attractive alternative route. Butanol (i.e. bio-butanol) can be produced from renewable resources by using similar fermentation methods as ethanol production. Acetone-butanol-ethanol (ABE) fermentation by *Clostridium acetobutylicum* is especially applied for formation of bio-butanol. Similar biomass feedstock as for ethanol production is used in ABE fermentation process as well.

Among various olefins, ethylene, propylene and butylenes are the most widely produced olefins in the chemical industry. So the direct conversion of (bio)-alcohols (methanol, ethanol and other alcohols) to these light olefins is of industrial importance. Production of light olefins from methanol has been reported in the literature (cf. B. V. Vora, T. L. Marker, P. T. Barger, H. E. Fullerton, H. P. Nilson, S. Kvisile. T. Fuglerud, Stud. Surf. Sci. Catal. 107 (1997) 87). Among various catalysts reported SAPO-34 was found to be the best with 100 % methanol conversion and 90 % of light olefins selectivity. On the other hand, SAPO-34 and microporous zeolites are known for the rapid deactivation by coke formation which eventually influences the methanol conversion as well olefins selectivity (A. J. Marchi and G. F. Froment, Appl. Catal. 71 (1991) 139). According to US 2006/0149109, a process converting mixed alcohols of methanol and ethanol feedstock to light olefins was reported. M. Inaba, K. Murata, M. Saito, I. Takahara, Green Chem., 9 (2007) 638 reported that Fe-H-ZSM-5 catalyst was found to be efficient for ethanol conversion into C3+ olefins. These authors also report a higher $C_3$ olefins production during ethanol conversion on Fe/H-ZSM-5 catalysts. US4698452 discloses the production of alkenes from a mixture of methanol/ethanol by dehydration at 300-450°C using a Zn or Mn-modified ZSM-5 catalyst. In this work, the authors identified the causes of deactivation as carbon deposition on the catalyst and collapse of zeolite framework. The selectivity of C3+ olefins was decreased from 31.1 % to 27.4 % over 900 min (15 h). The highest selectivity of propylene was about 12.8% with C3+ olefins selectivity of 28.7%. In EP 1 953 129 A1, ethanol and butanol were used separately to produce ethylene and butylenes in the first step and propylene via metathesis in a second step. Effect of mesoporosity in the propylene production from methanol was described in C. Mei, P. Wen, Z. Liu, H. Liu, Y. Wang, W. Yang, Z. Xie, W. Hua and Z. Gao, J. Catal. 258 (2008) 243. Enhancement in the propylene production was attributed to the newly generated mesopores together with low Bronsted acidity. However, no such a study was undertaken for the conversion of other alcohols into olefins, especially to improve the propylene and C3+ olefins selectivity. Also Song et al. (Catal. Lett (2009) 131:364-369) report about the production of propylene from ethanol over ZSM-5 zeolites, wherein the zeolites may be metal-modified.

It is therefore an object of the present invention to overcome the above disadvantages and to provide an improved method for producing alkenes, in particular propylene.

## SUMMARY OF THE INVENTION

The invention provides a method of producing alkenes by dehydration of a mixture of alcohols in a single step comprising

- providing two or more alcohols;
- contacting the mixture with a catalyst, wherein the catalyst is a metal-modified zeolite; wherein the metal-modified zeolite is mesoporous, H-ZSM5 modified with Zr zeolite and wherein the Zr-loading is 20 wt%;
- reacting the mixture in the temperature range of 350 to 500 °C, thereby producing the alkenes.

The invention will be better understood with reference to the detailed description when considered in conjunction with the help of the figures illustrated in the attached drawings.

## BRIEF DESCRIPTION OF THE DRAWINGS

FIG. 1 depicts the selectivity towards propylene during the conversion of ethanol/butanol mixtures into light olefins over 20% Zr-ZSM-5. The effect of temperature on the propylene selectivity is shown.
FIG. 2 depicts the conversion of ethanol/butanol mixtures into light olefins over 20% Zr-ZSM-5 at 450 °C. Propylene and C3+ olefins selectivity vs. time on stream.
FIG. 3 depicts the conversion of ethanol/butanol mixtures into light olefins over 20% Zr-mesoporous ZSM-5 at 400 °C. Propylene and C3+ olefins selectivity vs. time on stream.
FIG. 4 depicts the propylene selectivity over Zr-modified non-meso and mesoporous H-ZSM-5 samples at various temperatures and Zr loadings.
FIG. 5 depicts the propylene and C3+ olefins selectivity in the conversion of ethanol/butanol into light olefins over a 5% P-modified 20% Zr-ZSM-5 at 400 °C.
FIG. 6 depicts the propylene and C3+ olefins selectivity in the conversion of ethanol/butanol mixtures into light olefins over 5% P-modified 20% Zr-ZSM-5 at 450 °C.

## DETAILED DESCRIPTION OF THE INVENTION

This present invention discloses a method of producing alkenes by dehydration of a mixture of alcohols in a single step. The process is highly selective in that C3+ olefins may be obtained in a yield of above about 50%.

In a single step according to the invention means that no intermediate products have to be isolated or that several components have to be produced in separate process steps. Thus, the present invention can be considered as "one pot" procedure, i.e. the catalyst and the alcohols are mixed and brought into contact with each other so that the reaction for producing alkenes can be started.

In one embodiment the present invention provides a method of producing alkenes by dehydration of a mixture of alcohols in a single step comprising: providing two or more alcohols; contacting the mixture with a catalyst, wherein the catalyst is a metal-modified zeolite; and reacting the mixture in the temperature range of 350 to 500 °C, thereby producing the alkenes.

In the invention, the zeolite is H-ZSM-5 zeolite. The expression "ZSM-5" is used interchangeably with the expression "H-ZSM-5" throughout this entire specification.

In the present invention the zeolite is a mesoporous zeolite. Generally, a mesoporous material is a material containing pores with diameters between about 2 and about 50 nm. The mesoporous zeolite, which is prepared by -alkaline post-treatment, degrades its crystallinity and physical strength due to desilication, the extraction of framework silicon. The removal of framework silicon also means change of chemical properties of zeolite, i.e. change of intrinsic characteristics of zeolite.

The zeolite catalyst of the present invention is used in a metal-modified form. "Metal-modified" in this respect means that the zeolite is contacted with a metal compound, wherein the respective metal is linked to the surface of the zeolite via (wet) impregnation, adsorption, ion-exchange method or an otherwise known procedure in the art. Generally, every procedure which may link the metal to the surface of the zeolite may be possible for the present invention. In the invention the metal is Zr.

The metal loading, i.e. the amount of the metal, attached to the zeolite structure, of the inventive catalyst is 20 wt.%.

In one embodiment, the metal-modified zeolite may be prepared by pretreating commercially available zeolite, such as $NH_4$-ZSM-5, at elevated temperatures for several hours. In one embodiment the elevated temperatures may be between about 400 to about 600 °C. For example, the temperature may be about 400 °C, about 425 °C, about 450 °C, about 475 °C, about 500 °C, about 525 °C, about 550 °C, about 575 °C or about 600 °C. The respective metal compound may be dissolved in DI water and reacted with the pretreated zeolite compound. In one embodiment of the invention H-ZSM-5 may be obtained by treating commercial $NH_4$-ZSM-5 at 500 °C for 6 h in air. 5 to 20 wt% of metal loading may be added to the H-ZSM-5 by dry impregnation method. For example, calculated amounts of $ZrO(NO_3)_2 \cdot 2H_2O$ may be dissolved in de-ionized water and H-ZSM-5 may be added to the resulting solution. The prepared sample may be dried at 120 °C and further calcined at 500 °C for 4 h.

In the invention, the zeolite compound is a mesoporous compound. Mesoporous zeolites, such as ZSM-5, were synthesized by treating ZSM-5 with, for example, 0.2 M NaOH at 80 °C for 2 h. The sample dried at 110 °C may be treated with 1 M $NH_4NO_3$ at 70 °C for 24 h. The prepared sample may be dried at 120 °C and further calcined at 500 °C for 4 h. The required amounts of the respective metal were impregnated on the resulting mesoporous ZSM-5 catalysts, for example in the same manner as described above. Other methods for preparing mesoporous zeolites are known in the art and may be suitable for the present invention.

In another embodiment of the present invention, the zeolite is further modified with a modifying agent. The modifying agent may be, but is not limited to, an oxide containing compound or an oxy acid. These compounds are believed to modify the surface acidity, which results in improved stability of the metal catalyst. However, some oxides may also enhance the oligomerization of light olefins to produce bulky molecules and thus the selectivity for alkene production may drop considerably. Also, some metal oxides may be accompanied with surface oxygen atoms which help in oxidizing the reactant or products resulting in non-selective product formation. Moreover, metal or metal oxides may leach from the surface after a period of time. These factors should be considered when selecting an oxide containing compound or oxy acid. Some examples of the modifying agent of the present invention include phosphate or sulfate compounds such as, for example, phosphoric acid or sulfuric acid, or a derivative thereof. The modifying agent may, for example be, but is not limited to, $H_3PO_4$, $H_2PO_4^-$, $PO_4^{2-}$, $PO_4^{3-}$, $H_2SO_4$, $HSO_4^-$, or $SO_4^{2-}$. In one embodiment the modifying agent may be phosphoric acid or a derivative thereof, for example as mentioned above. In another embodiment, ammonium dihydrogen phosphate can be used to modify the catalyst. In another embodiment of the present invention the modifying agent may be, but is not limited to, tungstate-type modifiers such as $HWO_4^{2-}$, $WO_3$, chromates such as $CrO_4^{2-}$, $Cr_2O_7^{2-}$, $Cr_3O_{10}^{2-}$ and $Cr_4O_{13}^{6-}$, molybdates such as $MoO_3$, $MoO_4^{2-}$, $Mo_2O_7^{2-}$, $Mo_7O_{24}^{2-}$ and $Mo_8O_{26}^{4-}$, heteropoly acids such as $H_3PMo_{12}O_{40}$, $H_3PW_{12}O_{40}$ and $H_4[W_{12}SiO_{40}]$, silisic acid ($H_4SiO_4$) or derivates, or triflic acid ($CF_3SO_3H$) or derivates.

The amount of modifying agent anchored and/or impregnated in the catalyst is not limited and suitable amounts of the modifying agent would be understood to and can be determined by those of ordinary skill in the art. In an embodiment of the invention, the amount of modifying agent anchored and/or impregnated in the catalyst may include, but is not limited to, more than about 0.1 wt%, about 1.0 wt%, about 5 wt%, about 10 wt%, about 15 wt%, about 20 wt%, about 30 wt%, about 40 wt% and more than about 50 wt%. In one embodiment, the amount of modifying agent anchored and/or impregnated in the catalyst may include, but is not limited to, less than about 50 wt%, about 25 wt%, about 20 wt%, about 15 wt%, about 10 wt% or about 5 wt%. For example, the modifying agent may be used in an amount between about 1 to about 50 wt%, about 1 to about 20 wt%, about 5 to about 50 wt%, about 5 to about 20 wt%, and including any specific value within these ranges, such as, for example, about 1 wt%, about 2 wt%, about 3 wt%, about 4 wt%, about 5 wt%, about 6 wt%, about 7 wt%, about 8 wt%, about 9 or about 10 wt%. The amounts of modifying agent refer to the wt% of modifying agent in the final catalyst.

In one embodiment the modifying agent may be added to the catalyst, for example by impregnating the zeolite catalyst with the modifying agent, for example phosphoric acid. In one embodiment, the impregnation method may be, but is not limited to, a dry impregnation method. The meaning of the expression "dry impregnation" would be understood to those of ordinary skill in the art. Dry impregnation may include impregnation which uses an amount of water which is less or greater than or equal to that required to fill the pores of the substrate. For example, phosphoric acid may be added in an amount of about 5.0 to about 20.0 wt%. The amount of phosphoric acid refers to the wt% of phosphoric acid in the zeolite. In a further embodiment, the process for preparing the catalyst may comprise treating the zeolite at an elevated temperature before adding the phosphoric acid or grinding and/or pelletizing the catalyst.

In one embodiment of the invention, the zeolite may have, a silica/alumina (Si/Al) ratio between 30 to 280, such as between 30 to 80 or 30 to 50. In one embodiment, the Si/Al ratio may be 20, 30, 40, 50, 60, 70, 80, 90, 100, 110, 120, 130, 140, 150, 160, 170, 180, 190, 200, 210, 220, 230, 240, 250, 260, 270 or 280, such as 20, 30, 40, 50, 60, 70 or 80 and including any specific value within these ranges. Any of the above given values may be the lower or upper limit of a suitable range for the Si/Al ratio in the present invention.

The average particle size of the catalyst is not limited but may depend on the type and size of the reactor used and may be modified to suit the particular reactor used. The skilled person will be fully aware of the suitable particle sizes of the catalyst. Thus, every particle size which may be suitable for the purpose of the present invention is possible.

Generally, the pore volume of the catalyst is not limited but may be adapted or chosen in view of the specific system used. Thus, every pore volume which may be suitable for the purpose of the present invention is possible. In one embodiment of the invention, the catalyst composition may have a pore volume between about 0.1 cc/g to about 0.25 cc/g, such as between about 0.12 cc/g to about 0.22 cc/g or between about 0.15 cc/g to about 0.20 cc/g. In one embodiment, the pore volume may be, but is not limited to, less than about 0.25 cc/g, less than about 0.24 cc/g, less than about 0.23 cc/g, less than about 0.22 cc/g, less than about 0.21 cc/g, less than about 0.20 cc/g, less than about 0.19 cc/g, less than about 0.18 cc/g, less than about 0.17 cc/g, less than about 0.16 cc/g, less than about 0.15 cc/g, less than about 0.14 cc/g, less than about 0.12 cc/g, less than about 0.11 cc/g, or less than about 0.10, and including any specific value within these ranges.

Generally, the surface area of the catalyst is not limited but may depend on the catalyst modification and/or treatment. Therefore, every surface area which may be suitable for the purpose of the present invention is possible. In one embodiment of the invention, the catalyst may have a surface area between about 50 to about 350 $m^2$/g, such as between about 75 to about 300 $m^2$/g or between about 100 to about 250 $m^2$/g. In one embodiment, the surface area may but, but is not limited to, about 50 $m^2$/g, about 75 $m^2$/g, about 100 $m^2$/g, about 125 $m^2$/g, about 150 $m^2$/g, about 175 $m^2$/g, about 200 $m^2$/g, about 225 $m^2$/g, about 250 $m^2$/g, about 275 $m^2$/g, about 300 $m^2$/g, about 325 $m^2$/g or about 350 $m^2$/g, and including any specific value within these ranges, wherein each value may represent the lower or upper limit of the possible surface area.

The catalyst of the present invention may be used to catalyze the conversion or dehydration of a mixture of alcohols to alkenes. In the context of the present invention "a mixture of alcohols" means that two or more alcohols are used. For example, two, three, four, five, six or more alcohols may be used. The present invention enables the formation of alkenes from a mixture of alcohols, wherein the prior art generally uses one single alcohol as starting point for the preparation. Using a mixture of alcohols in combination with the catalyst of the present invention facilitate the production of a specific olefin or olefin mixtures. For example, starting from a mixture of butanol and ethanol, the inventive process leads to the formation of propylene and C3+ olefins with an alcohol conversion and olefin selectivity as described below.

The alcohols which may be used in the present invention may be, but are not limited to, alkyl alcohols. The alcohols may, for example be $C_{1-6}$ alkyl alcohols. The $C_{1-6}$ alkyl group of an $C_{1-6}$ alkyl alcohol may be, any straight or branched alkyl, for example, methyl, ethyl, n-propyl, iso-propyl, sec-propyl, n-butyl, iso-butyl, sec-butyl, t-butyl, n-pentyl, iso-pentyl, sec-pentyl, t-pentyl, n-hexyl, iso-hexyl, 1,2-dimethylpropyl, 2-ethylpropyl, 1-methyl-2-ethylpropyl, l-ethyl-2-methylpropyl, 1,1,2-trimethylpropyl, 1,1,2-triethylpropyl, 1,1-dimethylbutyl, 2,2-dimethylbutyl, 2-ethylbutyl, 1,3-dimethylbutyl, 2-methylpentyl or 3-methylpentyl. In one embodiment, the alcohols may be, but are not limited to, methanol, ethanol, n-butanol, 2-butanol, iso-butanol, 1-pentanol, 2-pentanol, 3-pentanol or 2-methyl-1-butan-1-ol. In one embodiment, the mixture of alcohols may be a combination of ethanol and butanol, such as a combination of ethanol and one or more of n-butanol, 2-butanol and iso-butanol. The alcohols in the mixture of alcohols may be used in an equal amount or one or more alcohols may be used in an excess compared to the other alcohol(s). In one embodiment the mixture of alcohols may be, for example, a 1:1 mixture of ethanol and butanol or mixtures of butanol. The alcohols may be used in a pure form.

In one embodiment of the invention, an aqueous solution of the alcohol mixture may be used. The concentration of the alcohols in the aqueous solution is not particularly limited, and suitable concentrations would be understood to and can be determined by those of ordinary skill in the art. In one embodiment, the concentration of the alcohols in the solution may be from 5 wt.% to 90 wt.%, such as 10 wt.% to 50 wt.%., based on the total alcohol concentration in the aqueous solution. For example, the concentration of the alcohols may be, 5 wt.%, 10 wt.%, 15 wt.%, 20 wt.%, 25 wt.%, 30 wt.%, 35 wt.%, 40 wt.%, 45 wt.%, 50 wt.%, 55 wt.%, 60 wt.%, 65 wt.%, 70 wt.%, 75 wt.%, 80 wt.%, 85 wt.%, or 90 wt.%. Each alcohol in the mixture may be present in the same amount as the other alcohols. In one illustrative embodiment of the invention, 5 wt.% butanol and 5 wt.% ethanol are used. In one embodiment of the invention each alcohol of the mixture may be present in a different amount and ratio.

The catalyst of the invention exhibits a high degree of alcohol conversion. For example, the alcohol conversion may be, but is not limited to, greater than about 25 %, greater than about 45 %, greater than about 50 %, greater than about 60%, greater than about 75 %, greater than about 85 %, greater than about 90 %, greater than about 95 %, greater than about 96 %, greater than about 97 %, greater than about 98 %, greater than about 99 % or even 100 %. Any specific value within these ranges lies within the scope of the present invention.

Suitable sources for the alcohol of the invention would be understood to or can be determined by those of ordinary skill in the art. For example, the alcohol may be obtained from a synthesis gas or biomass. In an embodiment, the alcohol may be, for example, and without limitation, obtained from biomass. The alcohol obtained from biomass may be referred to as bio-fuel or bio-alcohol. Bio-fuel or bio-alcohol, unlike petroleum, is a form of renewable energy that can be produced from agricultural feedstock. It may be produced from, but is not limited to, plant material, for example, very common crops such as sugar cane, potato, manioc and maize or even cellulosic materials, such as trees and grasses.

In the present invention, alkenes are produced from a mixture of alcohols. The alkenes which may be prepared may be $C_{2-6}$ alkenes. In one embodiment, the $C_{2-6}$ alkene may be, but are not limited to, any straight or branched alkene, such as ethylene, propylene, but-1-ene, cis-but-2-ene, trans-but-2-ene, isobutylene, pent-1-ene, cis-pent-2-ene, trans-pent-2-ene, 2-methylbut-1-ene, isopentene or 2-methylbut-2-ene.

In one embodiment, the present invention selectively produces propylene and C3+ olefins. The term "C3+ olefins" refers to alkenes having three or more carbon atoms. The selectivity of the present invention leads to formation of propylene in an amount of between about 30 to about 60%, such as about 30% to about 50%, about 30 to about 45% or about 30 to about 40%, based on the total amount of the alkenes produced. The selectivity of the process of the invention may be between about 31 to about 40%, about 31 to about 40%, about 32 to about 40%, about 33 to about 40%, about 34 to about 40%, about 34 to about 40% or about 35 to about 40%. In one embodiment of the present invention the propylene selectivity is up to about 45%, about 44%, about 43%, about 42%, about 41%, about 40%, about 39%, about 38%, about 37%, about 36%, about 35%, about 34%, about 33%, about 32%, about 31%, or about 30.

In one embodiment the total olefin content based on the total amount of products of the inventive process may be, but is not limited to, more than about 80 mol%, more than about 85 mol%, more than about 90 mol%, more than about 95 mol%, more than about 96 mol%, more than about 97 mol%, more than about 98 mol%, more than about 99 mol% or even 100 %. For example, the total olefin yield may be, but is not limited to, between about 80 to about 99 mol%, between about 85 to about 99 mol%, between about 90 to about 99 mol%, between about 95 to about 99 mol% or between about 98 to about 99 mol%, and including any specific value within these ranges.

The reaction temperature is between 300 to 500 °C, such as between 350 to 500 °C or 350 to 450 °C. For example, the reaction temperature may be between 400 to 450 °C. In one embodiment, the reaction temperature may be, 350 °C, 360 °C, 370 °C, 380 °C, 390 °C, 400 °C, 410 °C, 420 °C, 430 °C, 440 °C, 450 °C, 460 °C, 470 °C, 480 °C, 490 °C or 500 °C. Each value may represent the lower or upper limit of the possible reaction temperature.

The time-on-stream defining the actual time that a unit is operating and producing a product is not particularly limited and suitable time-on-stream would be understood to and can be determined by those of ordinary skill in the art.

In one embodiment, the dehydration reaction may be, for example, and without limitation, conducted in the gas or vapour phase. The dehydration reaction could also be conducted in the liquid phase, depending on the alcohol mixture and other reaction conditions.

The pressure of the dehydration reaction is not particularly limited, and suitable pressures would be understood to and can be determined by those of ordinary skill in the art. In one embodiment, the dehydration reaction may be, but is not limited to, conducted at a total pressure of about 1 bar to about 20 bar, such as about 5 bar to about 20 bar or 10 bar to about 20 bar or about atmospheric pressure.

The reactor type which may be used in the present invention is not particularly limited, and suitable reactor types would be understood to and can be determined by those of ordinary skill in the art. The reactor may have a simple design or may be complex. The type of reactor may be chosen by the skilled person depending on the components used in the process and the products which should be specifically obtained. In one embodiment, the dehydration reaction may be, but not limited to, conducted in a flow reactor or in a fluid or fluidized bed reactor. The flow reactor is not particularly limited, and suitable flow reactors would be understood to and can be determined by those of ordinary skill in the art. In one embodiment, the flow reactor may be a fixed bed reactor, a up flow fixed bed reactor, or a down flow fixed bed reactor. The fixed bed reactor may be isothermal and/or adiabatic.

In one embodiment, the process of the present invention may be carried out in a reactor having three zones. The first zone may be loaded with a suitable packaging material of particular size, such as glass bead, such as about 1 mm, about 2 mm, about 3 mm, about 4 mm or about 5 mm size, acting as pre-heating as well as mixing zone of the alcohol feedstock. Thus, generally the first zone may be suitable for pre-heating and mixing the system, i.e. the respective components. The second zone may be loaded with the catalyst where the vaporized feed enters in contact with the catalyst. The third zone is the post reaction zone. The reaction temperatures of the three zones were maintained by a three-heating zones heaters and the catalyst temperature was monitored using a thermocouple inside the catalytic bed. In one embodiment, a specific amount of the catalyst may be diluted with equal amount of quartz and loaded into a down flow fixed bed stainless steel reactor. Prior to the reaction, the catalysts were in situ treated under $N_2$ gas at a given temperature of about 400 °C, or 500 °C for a specific time, such as about 3h, about 4h, about 5h or about 6h. In one embodiment of the invention, the conversion of the mixture of alcohols is carried out in a continuous fixed bed flow reactor at low pressure. The temperature in the respective zones may be monitored. In one embodiment, several thermocouples are required. Depending on the reactor size the above general requirements may be adapted according to the knowledge of the skilled person.

The liquid hourly space velocity (LHSV, h$^{-1}$) of the dehydration reaction is not particularly limited and suitable liquid hourly space velocities would be understood to and can be determined by those of ordinary skill in the art. The expression "liquid hourly space velocity" defines the liquid volume of alcohol reactants supplied per hour divided by the volume of catalyst used. Generally, in case the LHSV increases, the selectivity of the reaction is decreased. In one embodiment, the dehydration reaction may be conducted with a liquid hourly space velocity of between 1.5 to 19 h$^{-1}$, such as 1.5 to 10 h$^{-1}$ or 1.5 to about 5 h$^{-1}$. In one embodiment, the LHSV may be, 1.5 h$^{-1}$, about 2 h$^{-1}$, about 2.5 h$^{-1}$, about 3 h$^{-1}$, about 3.5 h$^{-1}$, about 4 h$^{-1}$, about 4.5 h$^{-1}$, about 5 h$^{-1}$, about 6 h$^{-1}$, about 8 h$^{-1}$, about 10 h$^{-1}$, about 12 h$^{-1}$, about 15 h$^{-1}$, about 17 h$^{-1}$ or 19 h$^{-1}$, and including any specific value within these ranges, wherein the above values may be the upper or lower limit of the suitable ranges. In one embodiment, the LHSV may influence the selectivity of the reaction process, i.e. for example the amount of propylene obtained.

It has been observed that a catalyst composition of the invention exhibited a high degree of stability during a dehydration reaction, including a catalyst composition where deactivation was not detected for longer than about 20 hours, longer than about 25 hours, longer than about 30 hours, longer than about 50 hours, longer than about 60 hours, longer than about 75 hours, longer than about 100 hours, longer than about 110 hours, longer than about 150 hours, longer than about 250 hours, and including for any specific value within these ranges, such as, for example, longer than about 110, about 150 or about 250 hours.

In order that the invention may be readily understood and put into practical effect, particular embodiments will now be described by way of the following non-limiting examples.

## ILLUSTRATIVE EXERIMENTAL EXAMPLES

Exemplary embodiments of methods according to the invention as well as of reactants and further processes that may be used are described in the following section and the appended figures. Most of the experiments were carried out in a fixed bed reactor using 500 mg of catalyst. Mixtures of ethanol (Fischer Scientific, HPLC grade) and n-butanol (Alfa Acer) were fed into the reactor with a fixed flow rate between 0-1 ml/min and preheated at 175 °C before entering into the reactor. The reaction was carried out in the temperature range between 300 to 500 °C under atmospheric pressure.

Catalytic tests were performed by injecting mixtures of ethanol and butanol and/or their diluted solutions using a HPLC infusion pump. Helium was used as diluent gas and supplied into the system employing mass flow controllers. The analysis of the reaction products along with the reactants was performed using an online GC-HP (6890) equipped with a CP-Porabond Q column for the FID detector and a Haysep D column for the TCD one. The condensable products were condensed at low temperatures and then analyzed using a GC-MS for product identification. The conversion of alcohols and selectivity to light olefins are calculated as follows:

$$X_{AlcOH_i}(\%) = \frac{N^{in}_{AlcOH_i} - N^{out}_{AclOH_i}}{N^{in}_{AlcOH_{in}}} * 100 \tag{1}$$

$$S_{olefin_i}(\%) = \frac{N_{olefin_i}}{\sum_{i=1}^{n} N_{product_i}} * 100 \tag{2}$$

$X_{AlcoOH_i}$ is the conversion in mole percentage of ethanol and butanol, $X_{EtOH}$ and $X_{ButOH}$, respectively. $N^{in}_{AlcOH_i}$ are the moles of alcohol "$i$" (ethanol or butanol) fed and $N^{out}_{AlcOH_i}$ the moles of alcohol "$i$" observed in the products. $S_{olefin_i}$ is the selectivity towards olefin "$i$" in mol %, $N_{olefin_i}$ are the moles of light olefin "$i$" observed in the products and

$$\sum_{i=1}^{n} N_{product_i}$$ the total moles of reaction products.

The gas phase catalytic results during the conversion of mixtures of ethanol and butanol on H-ZSM-5-based catalysts are presented in the following examples. The reaction was carried out at between 350 to 500 °C and 1 atmospheric pressure using 500 mg of catalyst. Ethanol and n-butanol conversions reached 100 % at 350 °C and remained unchanged with further increase of temperature.

Catalytic results on H-ZSM-5 using pure ethanol yielded 12 % C3+ olefins with around 3 % of propylene, while using ethanol + butanol mixtures about 15 % propylene and 24 % of C3+ olefins were obtained. Using pure ethanol, 5 wt% $H_3PO_4$ on ZSM-5 catalyst yielded 15 wt% propylene, 20 wt% of butylenes and C3+ olefins selectivity of 43 % at 400 °C. Higher contents of $H_3PO_4$ (above 5%) resulted in higher ethylene selectivity, suppressing C3+ olefins.

The above results clearly suggest that there is urgent need for developing solid catalyst to selectively produce propylene and C3+ olefins. The present invention is particularly advantageous since, using diluted ethanol and butanol, high propylene and C3+ yield can be achieved on metal modified ZSM-5 catalyst using mixtures of bio-alcohols as feedstock. Examples 3, 4, and 7-11 are to be considered as comparative examples, since not fullfilling the required Zr loading of 20 wt.%..

**Example 1:**

Catalytic tests were performed as described above using Zr-modified ZSM-5 catalysts with Zr loadings from 5.0 to 20.0 wt%, prepared via wet impregnation method. All the catalysts were tested in the temperature range of 350 to 500 °C and the optimum temperature for high propylene yield as well as C3+ olefins yield was observed at 450 °C using a 5% ethanol and 5% butanol aqueous solution. The propylene yield increases when the Zr loading is increased from 5 % to 10 % and. Further increase in Zr loading has not shown any change in the propylene selectivity. The optimum content of Zr on ZSM-5 was found to be 20 wt%, leading to high propylene and C3+ selectivity as well as no formation of other products than olefins and paraffins. The results are shown in Table 1.

Table 1: Effect of Zr content on olefin selectivity during the conversion bio-ethanol/bio-butanol mixtures into light olefins at 450 °C

|  | Ethylene | Propylene | Butenes | n-butanol conversion % | ethanol conversion % | olefins selectivity % | paraffin selectivity 5 |
|---|---|---|---|---|---|---|---|
| 5% Zr/ZSM-5 | 37 | 34 | 12 | 100 | 100 | 84 | 12 |
| 10% Zr/ZSM-5 | 44 | 34 | 9.8 | 100 | 100 | 89.5 | 9.5 |
| 20 Zr/ZSM-5 | 44 | 34 | 10 | 100 | 100 | 90 | 10 |

**Example 2:**

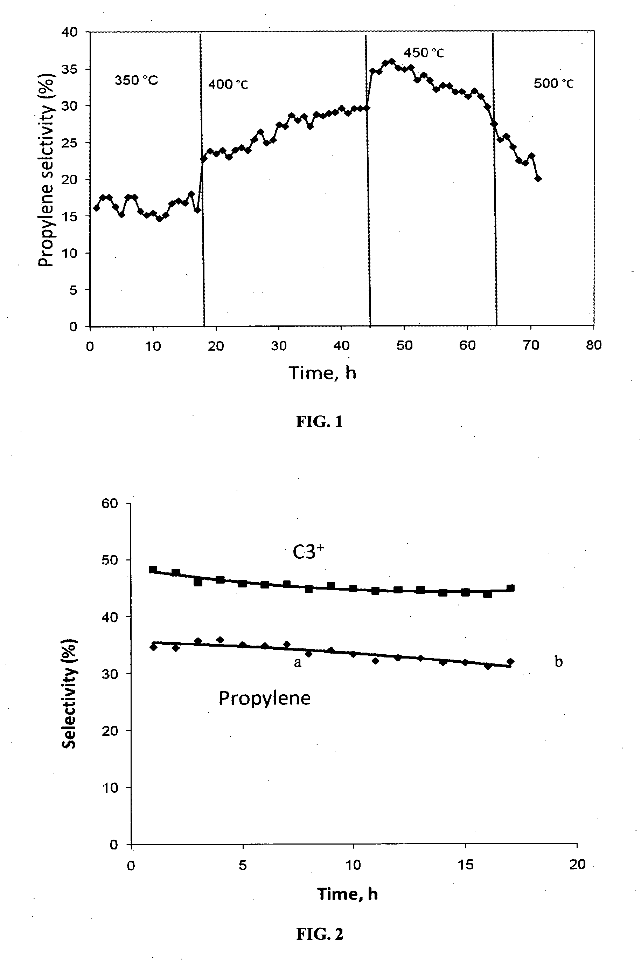

In a typical example, the results obtained on 20 wt% Zr/H-ZSM-5 catalyst in the temperature range between 300-500 °C are presented in Table 2. The propylene selectivity increases with reaction temperature, reaching a maximum of 34 % at 450 °C. Further increase in temperature decreases the propylene yield. On the other hand, butylenes selectivity decreases with temperature until 450 °C and shows a slight increase at 500 °C. At lower temperatures, paraffin selectivity is higher and decreases as temperature is increased. Figure 1 shows the selectivity towards propylene during the conversion of ethanol/butanol mixtures at different temperatures.

Table 2: Effect of the reaction temperature on the catalytic performance in the conversion of bio-ethanol/bio-butanol mixtures into light olefins over 20 wt% Zr/ZSM-5 catalyst

| Reactor temperature | Propylene selectivity | Ethylene selectivity | Butene selectivity | Olefin selectivity | Paraffin selectivity | Butanol conversion | Ethanol conversion |
|---|---|---|---|---|---|---|---|
| 350 | 16 | 12 | 26 | 63 | 20 | 100 | 100 |
| 400 | 28 | 23 | 19 | 74 | 19 | 100 | 100 |
| 450 | 34 | 44 | 10 | 90 | 10 | 100 | 100 |
| 500 | 25 | 49 | 14 | 89 | 11 | 100 | 100 |

**Example 3:**

Specifically, the process of the present invention is aimed to convert aqueous solutions ethanol/butanol mixtures. In this regard we studied the effect of the concentration of alcohols in water from 10 to 90 wt %. The results are shown in Table 3. A 10% alcohol concentration yields the highest propylene selectivity. Higher alcohol concentrations result in a drop in propylene selectivity, although a propylene selectivity of around 25 % can still be achieved. This result is significant in the sense that the developed catalysts are able to tolerate the presence of water without losing performance.

Table 3: Effect of alcohol concentration on the catalytic performance in the conversion of bio-ethanol/bio-butanol mixtures into light olefins at 450 °C over 10 % Zr/ZSM-5 (80)

| wt% of alcohols in water | Selectivity (%) | | | | | Conversion (%) | |
|---|---|---|---|---|---|---|---|
| | Propylene | Ethylene | Butenes | Olefins | Paraffin | Butanol | Ethanol |
| 5 wt.% BuOH + 5 wt.% EtOH | 36 | 46 | 9 | 94 | 6 | 100 | 100 |
| 25 wt.% BuOH + 25 wt.% EtOH | 24 | 41 | 14 | 83 | 16 | 100 | 100 |
| 45 wt.% BuOH + 45 wt.% EtOH | 25 | 35 | 14 | 80 | 18 | 100 | 100 |

**Example 4:**

Catalytic tests under the similar reaction conditions as previously described were carried out varying the space velocity (LHSV) of the ethanol and butanol from 1.5 to 4.5 h$^{-1}$. 100 % conversion of ethanol and butanol is obtained in the whole LHSV range used in this invention (cf. Table 4). However, the selectivity towards propylene and C3+ olefins is decreased at high LHSV (4.5 h$^{-1}$) while the butenes selectivity is increased. Paraffin selectivity is also increased at high LHSVs.

Table 4: Effect of LHSV (h$^{-1}$) on the catalytic performance in the conversion of bioethanol/bio-butanol mixtures into light olefins at 450 °C over 10 % Zr/ZSM-5 (80).

| LHSV, h$^{-1}$ | Selectivity (%) | | | | | Conversion (%) | |
|---|---|---|---|---|---|---|---|
| | Propylene | Ethylene | Butenes | Olefins | Paraffins | Butanol | Ethanol |
| 1.5 | 36 | 51 | 8 | 95 | 5 | 100 | 100 |
| 3.0 | 36 | 46 | 9 | 94 | 6 | 100 | 100 |
| 4.5 | 29 | 46 | 12 | 84 | 10 | 100 | 100 |

**Example 5:**

20 wt% Zr on mesoporous H-ZSM-5 was found to be similarly active for the conversion of ethanol/butanol mixtures with high selectivity towards propylene and C3+ olefins. However, the major difference being a slightly lower optimum reaction temperature, 400 °C instead of 450 °C (Table 5).

Table 5: Effect of the reaction temperature on the catalytic performance in the conversion of bio-ethanol/bio-butanol mixtures into light olefins over 20 wt% Zr on mesoporous H-ZSM-5.

| Reactor temperature °C | Selectivity (%) | | | | | Conversion (%) | |
|---|---|---|---|---|---|---|---|
| | Propylene | Ethylene | Butenes | Olefins | Paraffins | Butanol | Ethanol |
| 350 | 25 | 36 | 18 | 85 | 13 | 100 | 100 |
| 400 | 35 | 44 | 12 | 90 | 9 | 100 | 100 |
| 450 | 31 | 48 | 11 | 91 | 9 | 100 | 100 |
| 500 | 18 | 49 | 20 | 86 | 14 | 100 | 100 |

**Example 6:**

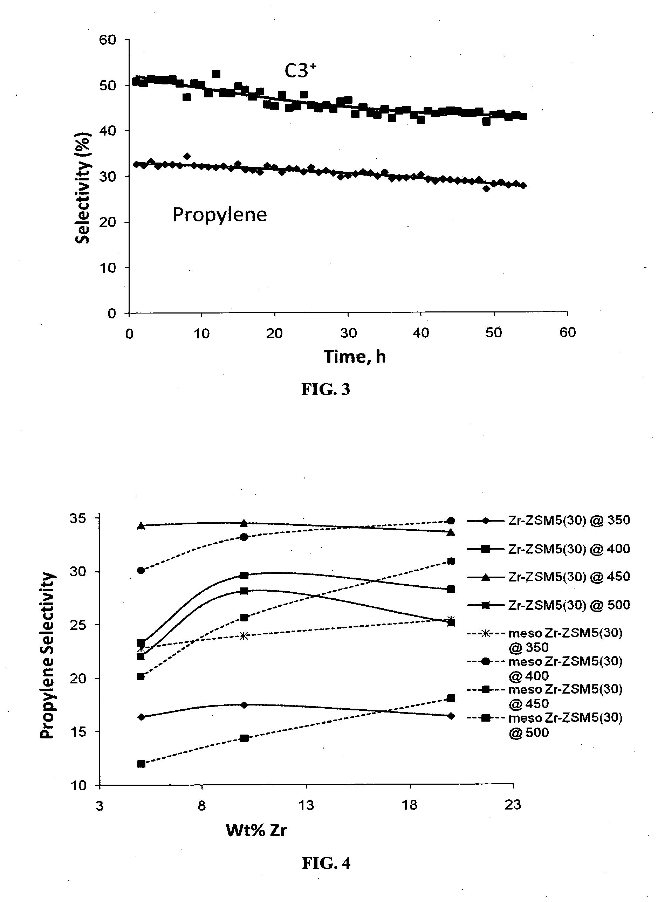

Propylene and C3+ selectivity as a function of time on stream experiment are shown in Figure 2 and 3 for 20 wt% Zr-modified non-meso and mesoporous HZSM-5, respectively. In both cases, a slight decrease in selectivity as a function of the time on stream is observed. In addition, Figure 4 shows the propylene selectivity of Zr-modified non-meso and mesoporous HZSM-5 samples at various temperatures and Zr loadings. It is clear that the optimum temperature is 450 °C for non-mesoporous ZSM-5 while for the mesoporous one is 400 °C. Regarding Zr loading, there is no significant effect for the non-mesoporous sample at the optimum temperature. However, the mesoporous sample at 400 °C shows a slight increase in propylene selectivity as the Zr loading is increased.

**Example 7:**

The influence of the $Si/Al_2$ ratio was systematically investigated. The results are presented in Table 6. The highest propylene selectivity was observed for $Si/Al_2$ ratio of 80 while the H-ZSM-5 sample with $Si/Al_2$ ratio of 280 shows the lowest propylene selectivity.

Table 6: Effect of $Si/Al_2$ ratio on the catalytic performance in the conversion of bio-ethanol/bio-butanol mixtures into light olefins.

| $Si/Al_2$ | Selectivity (%) | | | | | Conversion (%) | |
|---|---|---|---|---|---|---|---|
| | Propylene | Ethylene | Butenes | Olefins | Paraffins | Butanol | Ethanol |
| 30 | 34 | 44 | 10 | 89 | 10 | 100 | 100 |
| 50 | 34 | 46 | 10 | 91 | 9 | 100 | 100 |
| 80 | 36 | 46 | 9 | 94 | 6 | 100 | 100 |
| 280 | 8 | 48 | 25 | 91 | 19 | 100 | 100 |

**Example 8:**

The influence of various metals (10 wt%) was investigated. Table 7 shows a summary of the results. It can be conclude that the propylene selectivity decrease as follows: 10wt% Ga-ZSM5(80) > 10wt% Fe-ZSM5(80) >10wt% La-ZSM5(80) >10wt% Ba-ZSM5(80). However, under the same conditions, the Zr-modified sample shows the highest selectivity.

Table 7: Effect of type of metal on the catalytic performance in the conversion of bio-ethanol/bio-butanol mixtures into light olefins.

| Catalyst | Selectivity (%) | | | | | Conversion (%) | |
|---|---|---|---|---|---|---|---|
| | Propylene | Ethylene | Butenes | Olefins | Paraffins | Butanol | Ethanol |
| 10 wt.% La-ZSM-5 (80) | 17 | 48 | 18 | 85 | 15 | 100 | 100 |

(continued)

| Catalyst | Selectivity (%) | | | | | Conversion (%) | |
|---|---|---|---|---|---|---|---|
| | Propylene | Ethylene | Butenes | Olefins | Paraffins | Butanol | Ethanol |
| 10 wt.% Fe-ZSM-5 (80) | 21 | 48 | 16 | 87 | 13 | 100 | 100 |
| 10 wt.% Ga-ZSM-5 (80) | 24 | 48 | 14 | 88 | 12 | 100 | 100 |
| 10 wt.% Ba-ZSM-5 (80) | 11 | 48 | 22 | 82 | 18 | 100 | 100 |

**Example 9: (comparative example)**

In order to study the influence of the type of zeolites, different zeolites (H-Y, H-Beta, H-MOR and H-FER) were used to prepare 10 wt.% Zr-modified samples. Tables 8 a-d summarize the catalytic results. Clearly, Zr-modified ZSM-5 exhibits the highest propylene and C3 + olefins selectivity as compared to any of the other zeolites investigated. Thus, the catalytic performance of this sample is, by far, the best one.

Table 8a: Catalytic performance in the conversion of bio-ethanol/bio-butanol mixtures into light olefins over 10 wt% Zr on H-Y catalyst.

| Reactor temperature °C | Selectivity (%) | | | | | Conversion (%) | |
|---|---|---|---|---|---|---|---|
| | Propylene | Ethylene | Butenes | Olefins | Paraffins | Butanol | Ethanol |
| 350 | 3 | 51 | 18 | 75 | 28 | 100 | 100 |
| 400 | 2 | 51 | 21 | 77 | 25 | 100 | 100 |
| 450 | 2 | 50 | 23 | 76 | 26 | 100 | 100 |
| 500 | 1 | 49 | 24 | 75 | 26 | 100 | 100 |

Table 8b: Catalytic performance in the conversion of bio-ethanol/bio-butanol mixtures into light olefins over 10 wt% Zr on H-BETA catalysts

| Reactor temperature °C | Selectivity (%) | | | | | Conversion (%) | |
|---|---|---|---|---|---|---|---|
| | Propylene | Ethylene | Butenes | Olefins | Paraffins | Butanol | Ethanol |
| 350 | 7 | 47 | 19 | 75 | 25 | 100 | 100 |
| 400 | 6 | 50 | 19 | 76 | 24 | 100 | 100 |
| 450 | 3 | 50 | 20 | 73 | 27 | 100 | 100 |
| 500 | 1 | 47 | 19 | 68 | 32 | 100 | 100 |

Table 8c: Catalytic performance in the conversion of bio-ethanol/bio-butanol mixtures into light olefins over 10 wt%
Zr on H-MOR (90) catalysts

| Reactor temperature °C | Selectivity (%) | | | | | Conversion (%) | |
|---|---|---|---|---|---|---|---|
| | Propylene | Ethylene | Butenes | Olefins | Paraffins | Butanol | Ethanol |
| 350 | 4 | 48 | 13 | 66 | 34 | 100 | 100 |
| 400 | 1 | 49 | 16 | 66 | 34 | 100 | 100 |
| 450 | 0 | 48 | 17 | 65 | 35 | 100 | 100 |
| 500 | 0 | 48 | 18 | 66 | 34 | 100 | 100 |

Table 8d: Catalytic performance in the conversion of bio-ethanol/bio-butanol mixtures into light olefins over 10 wt%
Zr on FER (20) catalysts

| Reactor temperature °C | Selectivity (%) | | | | | Conversion (%) | |
|---|---|---|---|---|---|---|---|
| | Propylene | Ethylene | Butenes | Olefins | Paraffins | Butanol | Ethanol |
| 350 | 8 | 48 | 23 | 81 | 19 | 100 | 100 |
| 400 | 6 | 48 | 27 | 81 | 19 | 100 | 100 |
| 450 | 3 | 48 | 29 | 80 | 20 | 100 | 100 |
| 500 | 2 | 48 | 29 | 80 | 20 | 100 | 100 |

**Example 10:**

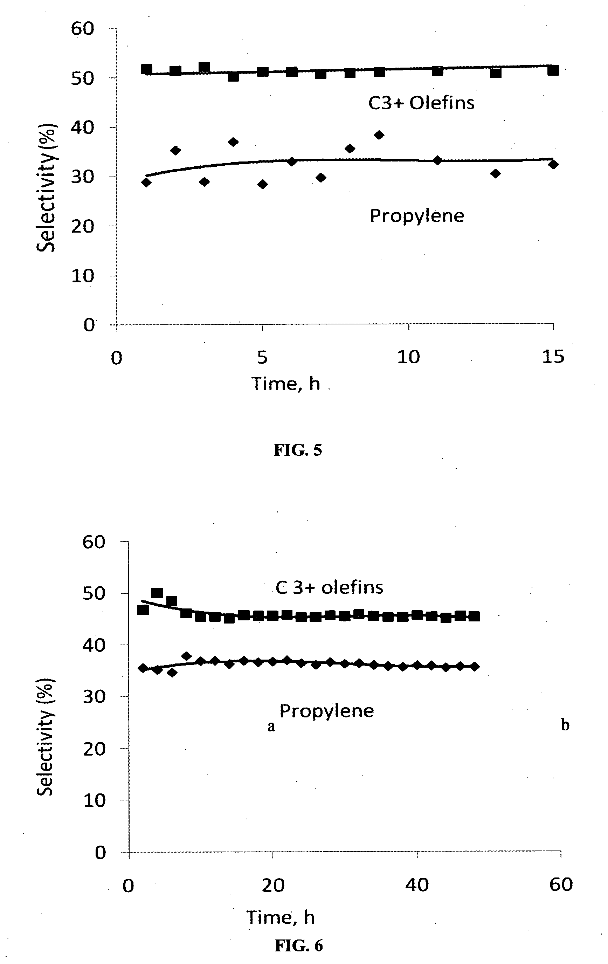

In order to obtain stable propylene and C3+ olefins selectivity during the conversion of bio-ethanol/bio-butanol mixtures into light olefins, P-modified Zr-ZSM-5 catalysts were synthesized using a sequential impregnation method. Thus, the 5 wt% P-modified catalysts show stable yield of propylene and C3+ olefins over 15 h at 400 °C (Figure 5) and 50 h at 450 °C (Figure 6).

**Example 11:**

Under similar reaction conditions previously described, a blank reactor without catalyst was tested at 400 °C. No significant conversion of either ethanol or butanol is observed during these tests.
It is clearly evident from the examples reported above that the process for selective production of C3+ olefins can be improved by adding metals such as Zr, Ga, La Fe, Li and elements such as P to H-ZSM-5 catalyst.

**Claims**

1. A method of producing alkenes by dehydration of a mixture of alcohols in a single step comprising:

   - providing two or more alcohols;
   - contacting the mixture with a catalyst, wherein the catalyst is a metal-modified zeolite, wherein the metal-modified zeolite is mesoporous, wherein the metal-modified zeolite is H-ZSM-5 modified with Zr, and wherein the Zr loading is 20 wt. %; and
   - reacting the mixture in a temperature range of 350 to 500 °C, thereby producing the alkenes.

2. The method of claim 1, wherein the two or more alcohols are selected from the group consisting of ethanol, propanol, n-butanol, 2-butanol, iso-butanol, 1-pentanol, 2-pentanol, 3-pentanol and 2-methyl-butan-1ol.

**3.** The method of claim 2, wherein the two or more alcohols are ethanol and butanol.

**4.** The method of claim 3, wherein the butanol is selected from the group of n-butanol, 2-butanol, iso-butanol and mixtures thereof.

**5.** The method of any of the preceding claims, wherein the alcohol is a bio-alcohol.

**6.** The method of any of the preceding claims, wherein an aqueous solution of the alcohol mixture is used.

**7.** The method of claim 6, wherein the alcohol concentration is from 5 wt.% to 90 wt.%, based on the total alcohol concentration in the aqueous solution.

**8.** The method of claim 7, wherein the alcohol concentration is from 10 wt.% to 50 wt.%, based on the total alcohol concentration in the aqueous solution.

**9.** The method of claim 8, wherein 5 wt.% ethanol and 5 wt.% butanol are used.

**10.** The method of any of the preceding claims, wherein the zeolite is further modified with a modifying agent.

**11.** The method of claim 10, wherein the modifying agent is phosphoric acid or a derivative thereof.

**12.** The method of any of the preceding claims, wherein the zeolite has a silica/alumina ratio between 30 to 280.

**13.** The method of any of the preceding claims, wherein the alkenes produced are ethylene, propylene and C3+ olefins.

**14.** The method of claim 13, wherein between 20 to 40% propylene, based on the total amount of the alkenes produced, is obtained.

**15.** The method of claim 14, wherein between 30 to 40% propylene, based on the total amount of the alkenes produced, is obtained.

**16.** The method of any of the preceding claims, wherein (1) the temperature is in a range of 400 to 450 °C; and/or (2) the Liquid Hourly Space Velocity (LHSV) is in the range of 1.5 to 9 h$^{-1}$.

**Patentansprüche**

**1.** Ein Verfahren zur Herstellung von Alkenen mittels Dehydrierung einer Alkoholmischung in einem einzigen Schritt, umfassend:

- Bereitstellen von zwei oder mehr Alkoholen;
- Kontaktieren der Mischung mit einem Katalysator, wobei der Katalysator ein metallmodifizierter Zeolith ist, wobei der metallmodifizierte Zeolith mesoporös ist, wobei der metallmodifizierte Zeolith H-ZSM-5, modifiziert mit Zr, ist und wobei die Zr-Ladung 20 Gew.-% beträgt; und
- Umsetzen der Mischung in einem Temperaturbereich von 350 bis 500 °C, wodurch die Alkene hergestellt werden.

**2.** Das Verfahren gemäß Anspruch 1, wobei die zwei oder mehr Alkohole ausgewählt sind aus der Gruppe bestehend aus Ethanol, Propanol, n-Butanol, 2-Butanol, iso-Butanol, 1-Pentanol, 2-Pentanol, 3-Pentanol und 2-Methylbutan-1-ol.

**3.** Das Verfahren gemäß Anspruch 2, wobei die zwei oder mehr Alkohole Ethanol und Butanol sind.

**4.** Das Verfahren gemäß Anspruch 3, wobei das Butanol ausgewählt ist aus der Gruppe von n-Butanol, 2-Butanol, iso-Butanol und Mischungen davon.

**5.** Das Verfahren gemäß einem der vorangegangenen Ansprüche, wobei der Alkohol ein Bioalkohol ist.

6. Das Verfahren gemäß einem der vorangegangenen Ansprüche, wobei eine wässrige Lösung der Alkoholmischung verwendet wird.

7. Das Verfahren gemäß Anspruch 6, wobei die Alkoholkonzentration von 5 Gew.-% bis 90 Gew.-%, bezogen auf die Gesamtalkoholkonzentration in der wässrigen Lösung, beträgt.

8. Das Verfahren gemäß Anspruch 7, wobei die Alkoholkonzentration von 10 Gew.-% bis 50 Gew.-%, bezogen auf die Gesamtalkoholkonzentration in der wässrigen Lösung, beträgt.

9. Das Verfahren gemäß Anspruch 8, wobei 5 Gew.-% Ethanol und 5 Gew.-% Butanol verwendet werden.

10. Das Verfahren gemäß einem der vorangegangenen Ansprüche, wobei der Zeolith ferner mit einem Modifiziermittel modifiziert ist.

11. Das Verfahren gemäß Anspruch 10, wobei das Modifiziermittel Phosphorsäure oder ein Derivat davon ist.

12. Das Verfahren gemäß einem der vorangegangenen Ansprüche, wobei der Zeolith ein Siliciumdioxid/Aluminiumoxid-Verhältnis zwischen 30 bis 280 aufweist.

13. Das Verfahren gemäß einem der vorangegangenen Ansprüche, wobei die hergestellten Alkene Ethylen, Propylen und C3+-Olefine sind.

14. Das Verfahren gemäß Anspruch 13, wobei zwischen 20 bis 40% Propylen, bezogen auf die Gesamtmenge der hergestellten Alkene, erhalten wird.

15. Das Verfahren gemäß Anspruch 14, wobei zwischen 30 bis 40% Propylen, bezogen auf die Gesamtmenge der hergestellten Alkene, erhalten wird.

16. Das Verfahren gemäß einem der vorangegangenen Ansprüche, wobei (1) die Temperatur in einem Bereich von 400 bis 450 °C liegt; und/oder (2) die stündliche Flüssigkeitsraumgeschwindigkeit (LHSV) im Bereich von 1,5 bis 9 h$^{-1}$ liegt.

**Revendications**

1. Procédé de production d'alcènes par déshydratation d'un mélange d'alcools en une seule étape, comprenant :

   - l'obtention de deux ou plus de deux alcools ;
   - la mise en contact du mélange avec un catalyseur, lequel catalyseur est une zéolite modifiée par un métal, laquelle zéolite modifiée par un métal est mésoporeuse, laquelle zéolite modifiée par un métal est la H-ZSM-5 modifiée par Zr, la charge de Zr étant de 20 % en poids ; et
   - la réaction du mélange à une température située dans la plage allant de 350 à 500°C, ce qui produit ainsi les alcènes.

2. Procédé selon la revendication 1, dans lequel les deux ou plus de deux alcools sont choisis dans l'ensemble constitué par l'éthanol, le propanol, le n-butanol, le 2-butanol, l'isobutanol, le 1-pentanol, le 2-pentanol, le 3-pentanol et le 2-méthylbutan-1-ol.

3. Procédé selon la revendication 2, dans lequel les deux ou plus de deux alcools sont l'éthanol et le butanol.

4. Procédé selon la revendication 3, dans lequel le butanol est choisi dans l'ensemble constitué par le n-butanol, le 2-butanol, l'isobutanol et leurs mélanges.

5. Procédé selon l'une quelconque des revendications précédentes, dans lequel l'alcool est un bioalcool.

6. Procédé selon l'une quelconque des revendications précédentes, dans lequel une solution aqueuse du mélange d'alcools est utilisée.

**7.** Procédé selon la revendication 6, dans lequel la concentration d'alcool est de 5 % en poids à 90 % en poids par rapport à la concentration d'alcool totale dans la solution aqueuse.

**8.** Procédé selon la revendication 7, dans lequel la concentration d'alcool est de 10 % en poids à 50 % en poids par rapport à la concentration d'alcool totale dans la solution aqueuse.

**9.** Procédé selon la revendication 8, dans lequel 5 % en poids d'éthanol et 5 % en poids de butanol sont utilisés.

**10.** Procédé selon l'une quelconque des revendications précédentes, dans lequel la zéolite est en outre modifiée par un agent modificateur.

**11.** Procédé selon la revendication 10, dans lequel l'agent modificateur est l'acide phosphorique ou un dérivé de celui-ci.

**12.** Procédé selon l'une quelconque des revendications précédentes, dans lequel la zéolite a un rapport silice/alumine compris entre 30 et 280.

**13.** Procédé selon l'une quelconque des revendications précédentes, dans lequel les alcènes produits sont l'éthylène, le propylène et des oléfines en C3+.

**14.** Procédé selon la revendication 13, dans lequel entre 20 et 40 % de propylène, par rapport à la quantité totale des alcènes produits, sont obtenus.

**15.** Procédé selon la revendication 14, dans lequel entre 30 et 40 % de propylène, par rapport à la quantité totale des alcènes produits, sont obtenus.

**16.** Procédé selon l'une quelconque des revendications précédentes, dans lequel (1) la température est située dans la plage allant de 400 à 450°C ; et/ou (2) la vitesse spatiale horaire de liquide (VSHL) est située dans la plage allant de 1,5 à 9 h$^{-1}$.

**FIG. 1**

**FIG. 2**

**FIG. 3**

**FIG. 4**

**FIG. 5**

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060149109 A
- US 4698452 A
- EP 1953129 A1

**Non-patent literature cited in the description**

- **H. ZIMMERMANN ; R. WALZI.** Ullmann's Encyclopedia of Industrial Chemistry. John Wiley & Sons, 2010
- **M. BAERNS ; O. BUYEVSKAYA.** *Catal. Today,* 1998, vol. 45, 13
- **B. V. VORA ; T. L. MARKER ; P. T. BARGER ; H. E. FULLERTON ; H. P. NILSON ; S. KVISILE. T. FUGLERUD.** *Stud. Surf. Sci. Catal.,* 1997, vol. 107, 87
- **A. J. MARCHI ; G. F. FROMENT.** *Appl. Catal.,* 1991, vol. 71, 139
- **M. INABA ; K. MURATA ; M. SAITO ; I. TAKAHARA.** *Green Chem.,* 2007, vol. 9, 638
- **C. MEI ; P. WEN ; Z. LIU ; H. LIU ; Y. WANG ; W. YANG ; Z. XIE ; W. HUA ; Z. GAO.** *J. Catal.,* 2008, vol. 258, 243
- **SONG et al.** *Catal. Lett,* 2009, vol. 131, 364-369